# EUROPEAN PATENT SPECIFICATION

(11) **EP 1 027 100 B1**
(45) Date of publication and mention of the grant of the patent: **05.03.2003**
(21) Application number: 98933068.3
(22) Date of filing: 02.07.1998
(51) Int. Cl.: A61N 1/362

(54) **AUGMENTATION OF ELECTRICAL CONDUCTION AND CONTRACTILITY BY BIPHASIC CARDIAC PACING**
VERBESSERUNG DER ELEKTRISCHEN REIZLEITUNG UND DES KONTRAKTIONSVERMÖGENS DES HERZENS DURCH ZWEIPHASENSTIMULATION
AUGMENTATION DE LA CONDUCTION ET DE LA CONTRACTILITE ELECTRIQUES PAR STIMULATION DIPHASIQUE DU COEUR

(43) Date of publication of application: 16.08.2000
(73) Proprietor: The Mower Family CHF Treatment Irrevocable Trust, Baltimore, MD 21218 (US)
(72) Inventor: Mower, Morton M., Baltimore, MD 21218 (US)
(74) Representative: Mohnhaupt, Dietrich
(86) International application number: US9813737
(87) International publication number: WO00001443

(56) References cited:
- EP-A- 0 491 649
- US-A- 4 343 312
- US-A- 4 402 322
- US-A- 4 543 956
- US-A- 4 903 700
- US-A- 5 601 608

## Description

### FIELD OF THE INVENTION

This invention relates generally to the stimulation of muscle tissue. In particular, this invention relates to an apparatus for cardiac stimulation and pacing with biphasic waveforms leading to improved conduction and contractility.

### BACKGROUND OF THE INVENTION

The function of the cardiovascular system is vital for survival. Through blood circulation, body tissues obtain necessary nutrients and oxygen, and discard waste substances. In the absence of circulation, cells begin to undergo irreversible changes that lead to death. The muscular contractions of the heart are the driving force behind circulation.

In cardiac muscle, the muscle fibers are interconnected in branching networks that spread in all directions through the heart. When any portion of this net is stimulated, a depolarization wave passes to all of its parts and the entire structure contracts as a unit. Before a muscle fiber can be stimulated to contract, its membrane must be polarized. A muscle fiber generally remains polarized until it is stimulated by some change in its environment. A membrane can be stimulated electrically, chemically, mechanically or by temperature change. The minimal stimulation strength needed to elicit a contraction is known as the threshold stimulus. The maximum stimulation amplitude that may be administered without eliciting a contraction is the maximum subthreshold amplitude.

Where the membrane is stimulated electrically, the impulse amplitude required to elicit a response is dependent upon a number of factors. First, is the duration of current flow. Since the total charge transferred is equal to the current amplitude times the pulse duration, increased stimulus duration is associated with a decrease in threshold current amplitude. Second, the percentage of applied current that actually traverses the membrane varies inversely with electrode size. Third, the percentage of applied current that actually traverses the membrane varies directly with the proximity of the electrode to the tissue. Fourth, the impulse amplitude required to elicit a response is dependent upon the timing of stimulation within the excitability cycle.

Throughout much of the heart are clumps and strands of specialized cardiac muscle tissue. This tissue comprises the cardiac conduction system and serves to initiate and distribute depolarization waves throughout the myocardium. Any interference or block in cardiac impulse conduction may cause an arrhythmia or marked change in the rate or rhythm of the heart.

Sometimes a patient suffering from a conduction disorder can be helped by an artificial pacemaker. Such a device contains a small battery powered electrical stimulator. When the artificial pacemaker is installed, electrodes are generally threaded through veins into the right ventricle, or into the right atrium and right ventricle, and the stimulator is planted beneath the skin in the shoulder or abdomen. The leads are planted in intimate contact with the cardiac tissue. The pacemaker then transmits rhythmic electrical impulses to the heart, and the myocardium responds by contracting rhythmically. Implantable medical devices for the pacing of the heart are well known in the art and have been used in humans since approximately the mid 1960s.

Either cathodal or anodal current may be used to stimulate the myocardium. However anodal current is thought not to be useful clinically. Cathodal current comprises electrical pulses of negative polarity. This type of current depolarizes the cell membrane by discharging the membrane capacitor, and directly reduces the membrane potential toward threshold level. Cathodal current, by directly reducing the resting membrane potential toward threshold has a one-half to one-third lower threshold current in late diastole than does anodal current. Anodal current comprises electrical pulses of positive polarity. The effect of anodal current is to hyperpolarize the resting membrane. On sudden termination of the anodal pulse, the membrane potential returns towards resting level, overshoots to threshold, and a propagated response occurs. The use of anodal current to stimulate the myocardium is generally discouraged due to the higher stimulation threshold, which leads to use of a higher current, resulting in a drain on the battery of an implanted device and impaired longevity. Additionally, the use of anodal current for cardiac stimulation is discouraged due to the suspicion that the anodal contribution to depolarization can, particularly at higher voltages, contribute to arrhythmogenesis.

Virtually all artificial pacemaking is done using stimulating pulses of negative polarity, or in the case of bipolar systems, the cathode is closer to the myocardium than is the anode. Where the use of anodal current is disclosed, it is generally as a charge of minute magnitude used to dissipate residual charge on the electrode. This does not affect or condition the myocardium itself. Such a use is disclosed in U.S. Patent No. 4,543,956 to Herscovici.

The use of a triphasic waveform has been disclosed in U.S. Patent Nos. 4,903,700 and 4,821,724 to Whigham et al., and U.S. Patent No. 4,343,312 to Cals et al. Here, the first and third phases have nothing to do with the myocardium per se, but are only envisioned to affect the electrode surface itself. Thus, the charge applied in these phases is of very low amplitude.

Lastly, biphasic stimulation is disclosed in U.S. Patent No. 4,402,322 to Duggan. The goal of this disclosure is to produce voltage doubling without the need for a large capacitor in the output circuit. The phases of the biphasic stimulation disclosed are of equal magnitude and duration.

Enhanced myocardial function is obtained through the biphasic pacing of the present invention. The combination of cathodal with anodal pulses of either a stimulating or conditioning nature, preserves the improved conduction and contractility of anodal pacing while eliminating the drawback of increased stimulation threshold. The result is a depolarization wave of increased propagation speed. This increased propagation speed results in superior cardiac contraction leading to an improvement in blood flow. Improved stimulation at a lower voltage level also results in reduction in power consumption and increased life for pacemaker batteries.

As with the cardiac muscle, striated muscle may also be stimulated electrically, chemically, mechanically or by temperature change. Where the muscle fiber is stimulated by a motor neuron, the neuron transmits an impulse which activates all of the muscle fibers within its control, that is, those muscle fibers in its motor unit. Depolarization in one region of the membrane stimulates adjacent regions to depolarize also, and a wave of depolarization travels over the membrane in all directions away from the site of stimulation. Thus, when a motor neuron transmits an impulse, all the muscle fibers in its motor unit are stimulated to contract simultaneously. The minimum strength to elicit a contraction is called the threshold stimulus. Once this level of stimulation has been met, the generally held belief is that increasing the level will not increase the contraction. Additionally, since the muscle fibers within each muscle are organized into motor units, and each motor unit is controlled by a single motor neuron, all of the muscle fibers in a motor unit are stimulated at the same time. However, the whole muscle is controlled by many different motor units that respond to different stimulation thresholds. Thus, when a given stimulus is applied to a muscle, some motor units may respond while others do not,

The combination of cathodal and anodal pulses of the present invention also provides improved muscular contraction where electrical muscular stimulation is indicated due to neural or muscular damage. Where nerve fibers have been damaged due to trauma or disease, muscle fibers in the regions supplied by the damaged nerve fiber tend to undergo atrophy and waste away. A muscle that cannot be exercised may decrease to half of its usual size in a few months. Where there is no stimulation, not only will the muscle fibers decrease in size, but they will become fragmented and degenerated, and replaced by connective tissue. Through electrical stimulation one may maintain muscle tone, such that upon healing or regeneration of the nerve fiber, viable muscle tissue remains.

Where muscle tissue has been damaged due to injury or disease, the regenerative process may be assisted by electrical stimulation. Enhanced muscle contraction is obtained through the biphasic stimulation of the present invention. The combination of cathodal with anodal pulses of either a stimulating or conditioning nature results in contraction of a greater number of motor units at a lower voltage level, leading to superior muscle response.

A device according to the preamble of claim 1 is disclosed in US-A-4 343 312.

### SUMMARY OF THE INVENTION

It is therefore an object of the present invention to provide improved stimulation of cardiac tissue.

It is another object of the present invention to increase cardiac output through superior cardiac contraction leading to greater stroke volume.

It is another object of the present invention to increase impulse propagation speed.

It is another object of the present invention to extend pacemaker battery life.

It is a further object of the present invention to obtain effective cardiac stimulation at a lower voltage level.

It is a further object of the present invention to eliminate the necessity of placing electrical leads in intimate contact with tissue to obtain tissue stimulation.

It is a further object of the present invention to provide improved stimulation of muscle tissue.

It is a further object of the present invention to provide contraction of a greater number of muscle motor units at a lower voltage level.

The above mentioned objects are achieved by an apparatus as defined in the independent claim 1.

Preferred or special embodiments of the apparatus are defined in the dependent claims.

According to the invention, the first and second phases have differing polarities, the two phases are of differing amplitude and of differing duration. In a preferred embodiment the first phase is in a chopped wave form, or the amplitude of the first phase is ramped. The first phase may be administered over 200 milliseconds post heart beat; i.e., greater than 200 milliseconds after the completion of a cardiac beating/pumping cycle. In a preferred alternative embodiment the first phase of stimulation is an anodal pulse at maximum subthreshold amplitude for a long duration, and the second phase of stimulation is a cathodal pulse of short duration and high amplitude. It is noted that the aforementioned alternative embodiments can be combined in differing fashions. It is also noted that these alternative embodiments are intended to be presented by way of example only, and are not limiting.

Pacemaker electronics needed to practice the present invention are well known to those skilled in the art. Current pacemaker electronics are capable of being programmed to deliver a variety of pulses, including those disclosed herein.

### BRIEF DESCRIPTION OF THE DRAWINGS

Fig. 1 is a schematic representation of leading anodal biphasic stimulation which is not in accordance with the invention.

Fig. 2 is a schematic representation of leading cathodal biphasic stimulation (not according to the invention).

Fig. 3 is a schematic representation of leading anodal stimulation of low level and long duration, followed by conventional cathodal stimulation.

Fig. 4 is a schematic representation of leading anodal stimulation of ramped low level and long duration, followed by conventional cathodal stimulation.

Fig. 5 is a schematic representation of leading anodal stimulation of low level and short duration administered in series, followed by conventional cathodal stimulation.

Fig. 6 graphs conduction velocity transverse to the fiber vs pacing duration resulting from leading anodal biphasic pulse.

Fig. 7 graphs conduction velocity parallel to the fiber vs pacing duration resulting from leading anodal biphasic pulse.

### DETAILED DESCRIPTION

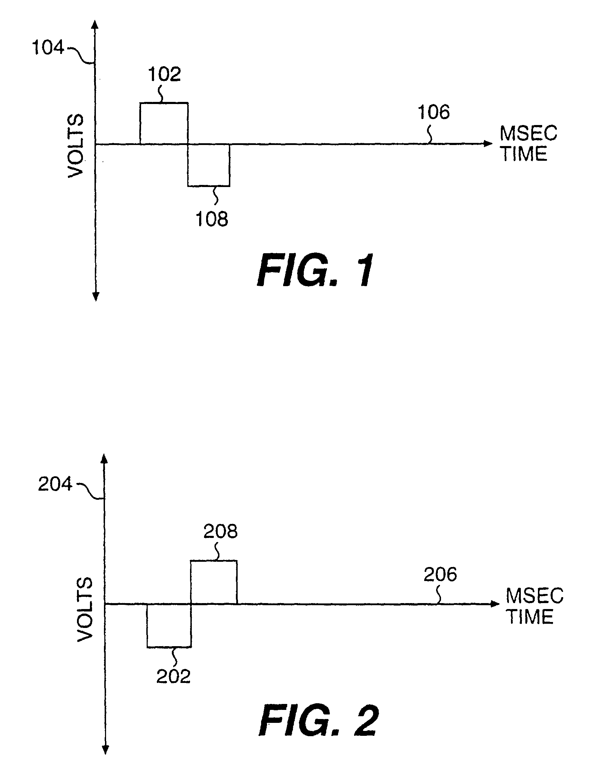

The present invention relates to the biphasic electrical stimulation of muscle tissue as defined in claim 1. **Figure 1** depicts biphasic electrical stimulation wherein a first stimulation phase comprising anodal stimulus **102** is administered having amplitude **104** and duration **106**. This first stimulation phase is immediately followed by a second stimulation phase comprising cathodal stimulation **108** of equal intensity and duration. This kind of stimulation is not claimed.

**Figure 2** depicts biphasic electrical stimulation wherein a first stimulation phase comprising cathodal stimulation **202** having amplitude **204** and duration **206** is administered. This first stimulation phase is immediately followed by a second stimulation phase comprising anodal stimulation **208** of equal intensity and duration. This stimulation is not comprised by the invention.

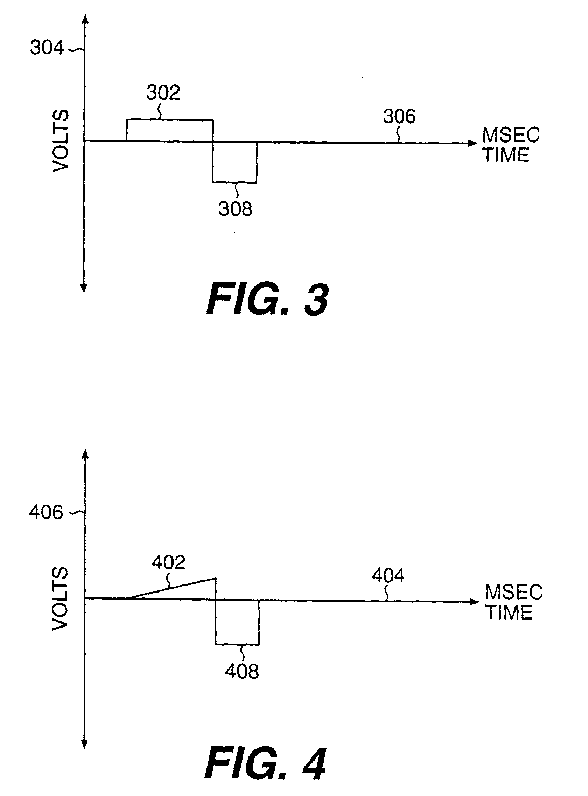

**Figure 3** depicts a stimulation according to the present invention, wherein a first stimulation phase comprising low level, long duration anodal stimulation **302** having amplitude **304** and duration **306** is administered. This first stimulation phase is immediately followed by a second stimulation phase comprising cathodal stimulation **308** of conventional intensity and duration. In a preferred embodiment of the invention, anodal stimulation **302** is at maximum subthreshold amplitude. In yet another preferred embodiment of the invention, anodal stimulation **302** is less than three volts. In another embodiment of the invention, anodal stimulation **302** is a duration of approximately two to eight milliseconds. In yet another alternative embodiment of the invention, cathodal stimulation **308** is of a short duration. In another alternative embodiment of the invention, cathodal stimulation **308** is approximately 0.3 to 0.8 milliseconds. In yet another alternative embodiment of the invention, cathodal stimulation **308** is of a high amplitude. In another alternative embodiment of the invention, cathodal stimulation **308** is in the approximate range of three to twenty volts. In yet another alternative embodiment of the present invention, cathodal stimulation **308** is of a duration less than 0.3 milliseconds and at a voltage greater than twenty volts. In another alternative embodiment, anodal stimulation **302** is administered over 200 milliseconds post heart beat. In the manner disclosed by these embodiments, as well as those alterations and modifications which may become obvious upon the reading of this specification, a maximum membrane potential without activation is achieved in the first phase of stimulation.

**Figure 4** depicts an alternative preferred embodiment of the present invention, wherein a first stimulation phase comprising anodal stimulation **402** is administered over period **404** with rising intensity level **406**. The ramp of rising intensity level **406** may be linear or non-linear, the slope may vary. This anodal stimulation is immediately followed by a second stimulation phase comprising cathodal stimulation **408** of conventional intensity and duration. In an alternative embodiment of the invention, anodal stimulation **402** rises to a maximum subthreshold amplitude. In yet another alternative embodiment of the invention, anodal stimulation **402** rises to a maximum amplitude that is less than three volts. In another alternative embodiment of the invention, anodal stimulation **402** is a duration of approximately two to eight milliseconds. In yet another alternative embodiment of the invention, cathodal stimulation **408** is of a short duration. In another alternative embodiment of the invention, cathodal stimulation **408** is approximately 0.3 to 0.8 milliseconds. In yet another alternative embodiment of the invention, cathodal stimulation **408** is of a high amplitude. In another alternative embodiment of the invention, cathodal stimulation **408** is in the approximate range of three to twenty volts. In yet another alternative embodiment of the present invention, cathodal stimulation **408** is of a duration less than 0.3 milliseconds and at a voltage greater than twenty volts. In another alternative embodiment, anodal stimulation **402** is administered over 200 milliseconds post heart beat. In the manner disclosed by these embodiments, as well as those alterations and modifications which may become obvious upon the reading of this specification, a maximum membrane potential without activation is achieved in the first phase of stimulation.

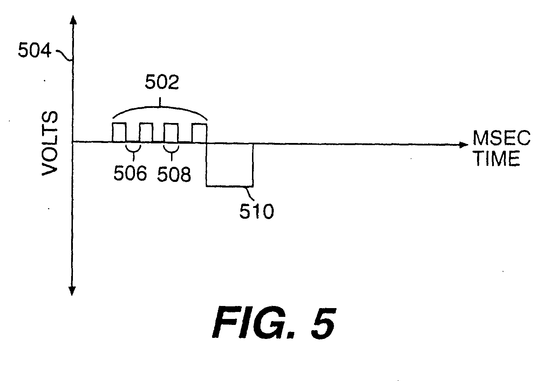

**Figure 5** depicts biphasic electrical stimulation wherein a first stimulation phase comprising series **502** of anodal pulses is administered at amplitude **504**. In one embodiment rest period **506** is of equal duration to stimulation period **508**, and is administered at baseline amplitude. In an alternative embodiment rest period **506** is of a differing duration than stimulation period **508** and is administered at baseline amplitude. Rest period **506** occurs after each stimulation period **508** with the exception that a second stimulation phase comprising cathodal stimulation **510** of conventional intensity and duration immediately follows the completion of series **502**. In an alternative embodiment of the invention, the total charge transferred through series **502** of anodal stimulation is at the maximum subthreshold level. In yet another alternative embodiment of the invention, the first stimulation pulse of series **502** is administered over 200 milliseconds post heart beat. In another alternative embodiment of the invention, cathodal stimulation **510** is of a short duration. In yet another alternative embodiment of the invention, cathodal stimulation **510** is approximately 0.3 to 0.8 milliseconds. In another alternative embodiment of the invention, cathodal stimulation **510** is of a high amplitude. In yet another alternative embodiment of the invention, cathodal stimulation **510** is in the approximate range of three to twenty volts. In another alternative embodiment of the invention, cathodal stimulation **510** is of a duration less than 0.3 milliseconds and at a voltage greater than twenty volts.

### EXAMPLE 1

Stimulation and propagation characteristics of the myocardium were studied in isolated hearts using pulses of differing polarities and phases. The experiments were carried out in five isolated Langendorff perfused rabbit hearts. Conduction velocity on the epicardium was measured using an array of bipolar electrodes. Measurements were made between six millimeters and nine millimeters from the stimulation site. Transmembrane potential was recorded using a floating intracellular microelectrode. The following protocols were examined: monophasic cathodal pulse, monophasic anodal pulse, leading cathodal biphasic pulse and leading anodal biphasic pulse.

Table 1 discloses the conduction speed transverse to fiber direction for each stimulation protocol administered, with stimulations of three, four and five volts and two millisecond pulse duration.

**TABLE 1**

| | Conduction Speed Transverse to Fiber Direction, 2 msec duration | | |
|---|---|---|---|
| | 3V | 4V | 5V |
| Cathodal Monophasic | 18.9 ± 2.5 cm/sec | 21.4 ± 2.6 cm/sec | 23.3 ± 3.0 cm/sec |
| Anodal Monophasic | 24.0 ± 2.3 cm/sec | 27.5 ± 2.1 cm/sec | 31.3 ± 1.7 cm/sec |
| Leading Cathodal Biphasic | 27.1 ± 1.2 cm/sec | 28.2 ± 2.3 cm/sec | 27.5 ± 1.8 cm/sec |
| Leading Anodal Biphasic | 26.8 ± 2.1 cm/sec | 28.5 ± 0.7 cm/sec | 29.7 ± 1.8 cm/sec |

Table 2 discloses the conduction speed along fiber direction for each stimulation protocol administered, with stimulations of three, four and five volts and two millisecond pulse duration.

**TABLE 2**

| | Conduction Speed Along Fiber Direction, 2 msec stimulation | | |
|---|---|---|---|
| | 3V | 4V | 5V |
| Cathodal Monophasic | 45.3 ± 0.9 cm/sec | 47.4 ± 1.8 cm/sec | 49.7 ± 1.5 cm/sec |
| Anodal Monophasic | 48.1 ± 1.2 cm/sec | 51.8 ± 0.5 cm/sec | 54.9 ± 0.7 cm/sec |
| Leading Cathodal Biphasic | 50.8 ± 0.9 cm/sec | 52.6 ± 1.1 cm/sec | 52.8 ± 1.7 cm/sec |
| Leading Anodal Biphasic | 52.6 ± 2.5 cm/sec | 55.3 ± 1.5 cm/sec | 54.2 ± 2.3 cm/sec |

The differences in conduction velocities between the cathodal monophasic, anodal monophasic, leading cathodal biphasic and leading anodal biphasic were found to be significant (p < 0.001). From the transmembrane potential measurements, the maximum upstroke ((dV/dt)max) of the action potentials was found to correlate well with the changes in conduction velocity in the longitudinal direction. For a four volt pulse of two millisecond duration, (dV/dt)max was 63.5 ± 2.4 V/sec for cathodal and 75.5 ± 5.6 V/sec for anodal pulses.

### EXAMPLE 2

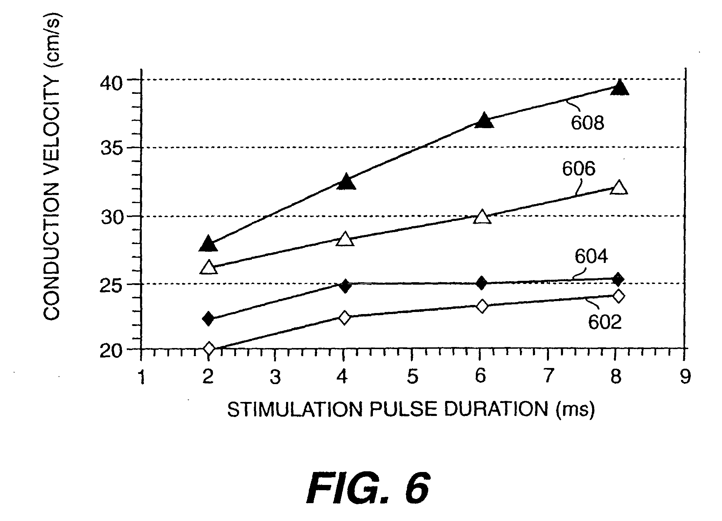

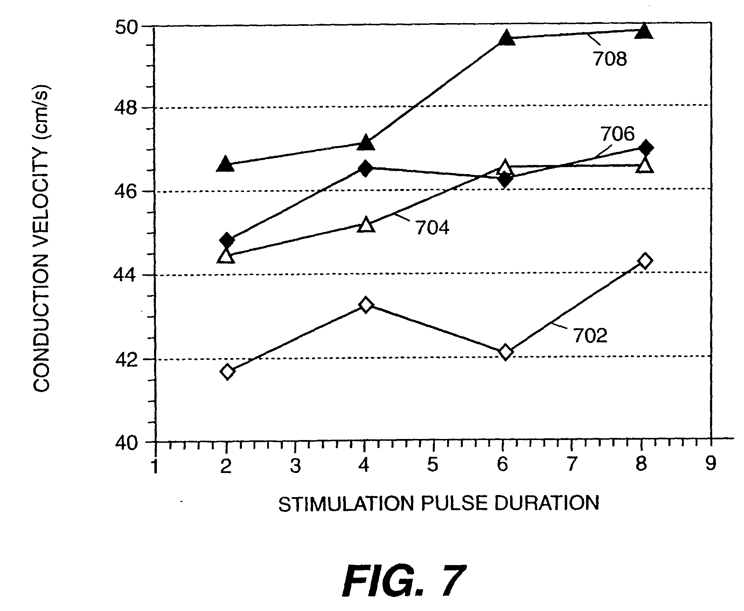

The effects of varying pacing protocols on the cardiac electrophysiology were analyzed using Langendorff prepared isolated rabbit hearts. Stimulation was applied to the heart at a constant voltage rectangular pulse. The following protocols were examined: monophasic anodal pulse, monophasic cathodal pulse, leading anodal biphasic pulse and leading cathodal biphasic pulse. Administered voltage was increased in one volt steps from one to five volts for both anodal and cathodal stimulation. Duration was increased in two millisecond steps from two to ten milliseconds. Epicardial conduction velocities were measured along and transverse to the left ventricular fiber direction at a distance between three to six millimeters from the left ventricular free wall. Figures 6 and 7 depict the effects of stimulation pulse duration and the protocol of stimulation administered on the conduction velocities.

**Figure 6** depicts the velocities measured between three millimeters and six millimeters transverse to the fiber direction. In this region, cathodal monophasic stimulation **602** demonstrates the slowest conduction velocity for each stimulation pulse duration tested. This is followed by anodal monophasic stimulation **604** and leading cathodal biphasic stimulation **606**. The fastest conduction velocity is demonstrated by leading anodal biphasic stimulation **608**.

**Figure 7** depicts the velocities measured between three millimeters and six millimeters parallel to the fiber direction. In this region, cathodal monophasic stimulation **702** demonstrates the slowest conduction velocity for each stimulation pulse duration tested. Velocity results of anodal monophasic stimulation **704** and leading cathodal biphasic stimulation **706** are similar, with anodal monophasic stimulation demonstrating slightly quicker speeds. The fastest conductive velocity is demonstrated by leading anodal biphasic stimulation **708**.

When using the apparatus according to the invention, electrical stimulation may be administered to the cardiac muscle. The anodal stimulation component of biphasic electrical stimulation augments cardiac contractility by hyperpolarizing the tissue prior to excitation, leading to faster impulse conduction, more intracellular calcium release, and the resulting superior cardiac contraction. The cathodal stimulation component eliminates the drawbacks of anodal stimulation, resulting in effective cardiac stimulation at a lower voltage level than would be required with anodal stimulation alone. This in turn, extends pacemaker battery life and reduces tissue damage.

In a second aspect, biphasic electrical stimulation may be administered to the cardiac blood pool, that is, the blood entering and surrounding the heart. This enables cardiac stimulation without the necessity of placing electrical leads in intimate contact with cardiac tissue.

In a third aspect, biphasic electrical stimulation may be applied to striated muscle tissue. The combination of anodal with cathodal stimulation results in the contraction of a greater number of muscular motor units at a lower voltage level, resulting in improved muscular response.

Having thus described the basic concept of the invention, it will be readily apparent to those skilled in the art that the foregoing detailed disclosure is intended to be presented by way of example only, and is not limiting. Various alterations, improvements and modifications will occur to those skilled in the art. Further, the pacing pulses described in this specification are well within the capabilities of existing pacemaker electronics with appropriate programming. Accordingly, the invention is limited only by the following claims.

## Claims

1. An apparatus for electrical cardiac pacing, the apparatus comprising means for applying electrical stimulation to cardiac tissue, and a pacing output circuitry connected to said applying means, the pacing output circuitry producing pulses to stimulate the cardiac tissue to effect cardiac pacing, the electrical waveform comprising application phases each having a phase polarity, a phase amplitude, a phase shape and a phase duration,
**characterised in that** the electrical waveform produces a biphasic cardiac pacing and consists of a first positive phase (302) for preconditioning the myocardium to accept subsequent stimulation, and an immediately following second, negative stimulation phase (308), the first phase duration (306) being greater than the second phase duration, and the first phase amplitude (304) being smaller than that of the second phase.

2. The apparatus of claim 1, wherein the first phase amplitude (406) is ramped from a baseline value to a second value.

3. The apparatus of claim 1, wherein the first, positive preconditioning phase is composed of a series (502) of positive pulses of a predetermined amplitude (504) and duration, each pulse of this series being followed by a rest period, said second, negative stimulating pulse immediately following the last of these positive pulses without any rest period.

4. The apparatus of claim 3, wherein each of said rest periods is of equal duration to each of said positive pulses in said series of pulses.

5. The apparatus of claim 1, wherein the amplitude of said first, positive phase is comprised between 0.5 and 3.5 volts.

6. The apparatus of claim 1, wherein the first phase duration is one to nine milliseconds.

7. The apparatus of claim 1, wherein the second phase duration is 0.2 to 0.9 milliseconds.

8. The apparatus of claim 1, wherein the amplitude of said second, negative phase is comprised between 2 and 20 volts.

9. The apparatus of claim 1, wherein the second phase duration is less than 0.3 milliseconds and the second phase amplitude is greater than 20 volts.

10. The apparatus of claim 1, wherein the first stimulation pulse is initiated more than 200 milliseconds after completion of the cardiac beating cycle.

## Patentansprüche

1. Elektrischer Herzschrittmacher-Apparat, mit Mitteln zum Anlegen elektrischer Stimulation an Herzgewebe, und mit einer Schrittmacher-Ausgangsschaltung, die mit den genannten Mitteln verbunden sind, wobei die Schrittmacher-Ausgangsschaltung Impulse zur Stimulierung des Herzgewebes und Erzielung einer Herzschrittmacher-Wirkung erzeugt und die elektrische Wellenform Applikationsphasen aufweist, von denen jede eine Phasenpolarität, eine Phasenamplitude, eine Phasenform und eine Phasendauer besitzt,
**dadurch gekennzeichnet, dass** die elektrische Wellenform eine Biphasen-Herzschrittmachung erzeugt und aus einer ersten positiven Phase (302) zur Vorkonditionierung des Myocards zwecks Akzeptanz der nachfolgenden Stimulierung und einer unmittelbar darauf folgenden zweiten, negativen Stimulierungsphase (308) besteht, wobei die Dauer (306) der ersten Phase grösser als die Dauer der zweiten Phase ist und die Amplitude (304) der ersten Phase kleiner als diejenige der zweiten Phase ist.

2. Apparat nach Anspruch 1, worin die Amplitude (406) der ersten Phase von einem Grundwert auf einen zweiten Wert ansteigt.

3. Apparat nach Anspruch 1, worin die erste, positive Vorkonditionierungsphase aus einer Reihe (502) von positiven Impulsen vorgegebener Amplitude (504) und Dauer zusammengesetzt ist und jeder einzelne Impuls dieser Serie von einer Ruheperiode gefolgt wird, und dass der genannte zweite, negative Stimulierungsimpuls unmittelbar auf den letzten dieser positiven Impulse ohne jeder Ruheperiode folgt.

4. Apparat nach Anspruch 3, worin jede der genannten Ruheperioden von der gleichen Dauer wie jeder der genannten positiven Impulse in der genannten Impulsserie ist.

5. Apparat nach Anspruch 1, worin die Amplitude der ersten, positiven Phase zwischen 0,5 und 3,5 V liegt.

6. Apparat nach Anspruch 1, worin die Dauer der ersten Phase eine bis neun Millisekunden beträgt.

7. Apparat nach Anspruch 1, worin die Dauer der zweiten Phase 0,2 bis 0,9 Millisekunden beträgt.

8. Apparat nach Anspruch 1, worin die Amplitude der zweiten, negativen Phase zwischen 2 und 20 V liegt.

9. Apparat nach Anspruch 1, worin die Dauer der zweiten Phase weniger als 0,3 Millisekunden beträgt und die Amplitude der zweiten Phase grösser als 20 V ist.

10. Apparat nach Anspruch 1, worin der erste Stimulationsimpuls mehr als 200 Millisekunden nach Vervollständigung des Herzschlagzyklus eingeleitet wird.

## Revendications

1. Appareil pour la stimulation cardiaque électrique, l'appareil comprenant des moyens pour appliquer une stimulation électrique au tissu cardiaque, et un circuit de sortie de stimulation relié auxdits moyens d'application, le circuit de sortie produisant des impulsions pour stimuler le tissu cardiaque afin d'effectuer une action de pacemaker, la forme d'onde électrique comprenant des phases d'application, chacune d'elles ayant une polarité de phase, une amplitude de phase, une forme de phase et une durée de phase,
**caractérisé en ce que** la forme d'onde électrique produit une stimulation cardiaque biphasé et consiste en une première phase positive (302) pour le pré-conditionnement du myocarde afin qu'il accepte une stimulation subséquente, et une seconde phase de stimulation négative (308) immédiatement suivante, la durée de la première phase (306) étant plus grande que la durée de la seconde phase, et l'amplitude de la première phase (304) étant plus petite que celle de la seconde phase.

2. Appareil selon la revendication 1, dans lequel l'amplitude de la première phase (406) va en augmentant à partir d'une valeur de base vers une seconde valeur.

3. Appareil selon la revendication 1, dans lequel la première phase positive de pré-conditionnement est composée d'une série (502) d'impulsions positives, chacune des impulsions de cette série étant suivie par une période de pause, ladite seconde impulsion de stimulation négative suivant immédiatement et sans période de pause à la dernière de ces impulsions positives.

4. Appareil selon la revendication 3, dans lequel chacune desdites périodes de pause a la même durée que chacune desdites impulsions positives dans ladite série d'impulsions.

5. Appareil selon la revendication 1, dans lequel l'amplitude de la première phase positive est comprise entre 0,5 et 3,5 volts.

6. Appareil selon la revendication 1, dans lequel la durée de la première phase est d'une à neuf millisecondes.

7. Appareil selon la revendication 1, dans lequel la durée de la seconde phase est de 0,2 à 0,9 milliseconde.

8. Appareil selon la revendication 1, dans lequel l'amplitude de la seconde phase négative est comprise entre 2 et 20 volts.

9. Appareil selon la revendication 1, dans lequel la durée de la seconde phase est plus courte que 0,3 milliseconde, et que l'amplitude de la seconde phase dépasse 20 volts.

10. Appareil selon la revendication 1, dans lequel la première impulsion de stimulation est initiée plus que 200 millisecondes après la fin du cycle de battement de coeur.
